# EUROPÄISCHE PATENTSCHRIFT

(11) **EP 1 144 001 B1**
(45) Veröffentlichungstag und Bekanntmachung des Hinweises auf die Patenterteilung: **21.05.2008**
(21) Anmeldenummer: 00903423.2
(22) Anmeldetag: 01.02.2000
(51) Int. Cl.: A61K 39/00

(54) **INAKTIVIERTE INFLUENZA-VIRUS-VAKZINE ZUR NASALEN ODER ORALEN APPLIKATION**
INACTIVATED INFLUENZA VIRUS VACCINE FOR NASAL OR ORAL ADMINISTRATION
VACCIN A VIRUS GRIPPAL INACTIVE POUR APPLICATION NASALE OU ORALE

(30) Priorität: 11.02.1999 AT 19499
(43) Veröffentlichungstag der Anmeldung: 17.10.2001
(73) Patentinhaber: Baxter Vaccine Aktiengesellschaft, 1221 Wien (AT)
(72) Erfinder: BARRETT, Noel, A-3400 Klosterneuburg/Weidling (AT); KISTNER, Otfried, A-1040 Wien (AT); GERENCER, Marijan, A-1170 Wien (AT); DORNER, Friedrich, A-1230 Wien (AT)
(74) Vertreter: Alge, Daniel
(86) Internationale Anmeldenummer: PCT/AT2000/000023
(87) Internationale Veröffentlichungsnummer: WO 2000/047222

(56) Entgegenhaltungen:
- WO-A-00/15251
- WO-A-94/19013
- WO-A-96/40290
- WO-A-97/49423
- WO-A-99/24068
- AVTUSHENKO S S ET AL: "Clinical and immunological characteristics of the emulsion form of inactivated influenza vaccine delivered by oral immunization" JOURNAL OF BIOTECHNOLOGY,NL,ELSEVIER SCIENCE PUBLISHERS, AMSTERDAM, Bd. 44, Nr. 1, 26. Januar 1996 (1996-01-26), Seiten 21-28, XP004036844 ISSN: 0168-1656
- GUEBRE-XABIER M. ET AL J. OF VIROLOGY Bd. 78, Nr. 14, Juli 2004, Seiten 7610 - 7618
- TREANOR J.J. ET AL J. OF CLINICAL MICROBIOLOGY Bd. 28, Nr. 3, M{rz 1990, Seiten 596 - 599
- GONZALEZ M. ET AL ARCH. DIS. CHILD. Bd. 83, 2000, Seiten 488 - 491

## Beschreibung

Die Erfindung betrifft eine Vakzin-Zusammensetzung, enthaltend mindestens ein inaktiviertes Influenzavirus-Antigen und Aluminium als Adjuvans zur nasalen oder oralen Anwendung für die Prophylaxe von Influenzavirus-Infektionen.

Influenza-Virusinfektionen stellen ein immer größeres gesundheitliches Risiko, insbesondere bei älteren Menschen und bei Personen mit chronischen Krankheiten, dar, da die Infektion bei diesen Personengruppen oftmals zu einer erhöhten Mortalitätsrate führt. Seit der Einführung einer inaktivierten Influenza-Vakzine, enthaltend inaktiviertes Virusmaterial aus infizierten bebrüteten Eiern, in den 40er Jahren ist das Risiko und der Verlauf der Infektion, sowie die Mortalitätsrate bei älteren Menschen gesunken.

Bisherige inaktivierte Influenzavirus-Vakzine aus Eiern sind zur parenteralen Verabreichung am Menschen lizensiert und induzieren anti-HA-IgG-Antikörper im Serum. Die Kreuz-Protektion gegen heterologe Influenzaviren ist bei einer natürlichen Infektion jedoch in erster Linie auf die Kreuzreaktivität von IgA-Antikörpern zurückzuführen (Liew et al., 1984, Eur. J. Immunol. 14: 350-356). Es wurde daher bei der Entwicklung von neuen Immunisierungsverfahren gegen Influenzavirus-Infektionen versucht, die Produktion der mukosalen IgA-Immunantwort zu stimulieren.

Dazu wurde eine Reihe von Entwicklungen zur intranasalen oder oralen Verabreichung von Influenzavirus-Vakzinen entwickelt. So führt etwa die Verabreichung einer inaktivierten Virus-Vakzine (Waldman et al., 1968, Nature 218:594-595) Avtushenko et al., 1996, J. of Biotechnology 44:21-28, einer inaktivierten Vakzine kombiniert mit Carboxyvinylpolymer (Oka et al., 1990, Vaccine 8:573-576) oder mit Pertussis-Toxin B Oligomer (Oka et al., 1994, Vaccine 12:1255-1258), einer Split-Virus-Vakzine mit Cholera-Toxin, E. coli hitze-labilem Enterotoxin oder Liposomen (Tamura et al., 1992, J. Immunol. 149:981-988, Komasse et al., 1998, Vaccine 16:248-254, de Haan, 1995, Vaccine 13:155-162), einer Emulsion-inaktivierten Vakzine (Avtushenko et al., 1996, J. Biotechnol. 44: 21-28) oder einer Kälte-adaptierten lebendattenuierten Influenzavirus-Vakzine (Belshe et al., 1998, N. Engl. J. Med. 338:1405-1412) nicht nur zur Induktion von HAI-IgG-Antikörpern im Serum sondern auch von sekretorischen IgA-Antikörpern der mukosalen Membran.

Inaktivierte Viren als oral oder nasal applizierte Vakzine müssen jedoch in hohen Konzentrationen gegeben werden, um zu einem signifikanten Antikörperanstieg zu führen. Die Verabreichung von inaktiviertem Influenzavirus oder Antigen in nebenwirkungsfreien und kommerziell vernünftigen Dosen führt bei nasaler oder oraler Verabreichung daher ohne die Zugabe eines Adjuvans nicht zu einer befriedigenden Immunantwort (Chen et al., 1989, Current Topics in Microbiology and Immunology: 146:101-106, Couch et al., 1997, J. Infect. Dis. 176:38-44). So ist etwa für die optimale Induktion der Immunantwort bei oraler Verabreichung einer Emulsion-inaktivierten Vakzine ein Antigengehalt zwischen 66 µg Antigen/Dosis und 384 µg Antigen/Dosis erforderlich (Avtushenko et al., 1996, J. Biotechnol. 44: 21-28). Diese Dosis liegt damit weit über der einer inaktivierten Vakzine für die parenterale Verabreichung, die bei etwa 15 µg Antigen/Dosis liegt.

Obwohl Cholera-Toxin, E. coli hitzelabiles Toxin und Pertussis-Toxin einen effektiven adjuvantierenden Effekt bei einer oralen oder nasalen Verabreichung von Influenza-Antigen aufweisen, werden sie aufgrund der toxischen Nebenwirkungen nicht für die humane Verwendung eingesetzt. Das einzige bisher zur Anwendung beim Menschen zugelassene Adjuvans ist Aluminium.

Eine in klinischen Studien befindliche Kälte-adaptierte, lebendattenuierte Influenzavirus-Vakzine für die nasale Verabreichung basiert auf Virus-Antigenen, von denen jährlich mittels genetischer Methoden Reassortanten hergestellt werden müssen, wobei die Gene für die Hämagglutinin- und Neuramidase-Antigene des entsprechenden Influenza A- bzw. B-Stammes auf einen attenuierten, kälte-adaptierten Mastervirusstamm übertragen werden. Diese Methode ist sehr zeitaufwendig und arbeitsintensiv. Zudem besteht die Gefahr, daß durch Reversion das attenuierte Virus zu einem virulenten Virus rückmutiert und damit eine Virämie auslösen kann. Bei Immunisierung mit Lebend-Viren erfolgt zudem eine weitere Ausbreitung im Körper des Geimpften. Auch ist bei Verwendung von kälte-adaptierten Viren immer die Notwendigkeit gegeben, die Virusvakzine unterhalb des Gefrierpunktes, möglichst bei -20°C aufzubewahren, was das unbedingte Einhalten der Kühlkette erforderlich macht, damit eine ausreichende Haltbarkeit für den Impfstoff gewährleistet ist.

Die Herstellung der Virus-Reassortanten und die Vermehrung der Impfviren erfolgt in Eiern, wodurch das Risiko besteht, dass in Eiern möglicherweise vorhandene verunreinigende, infektiöse Agenzien übertragen werden. Die Reinigung von Lebend-Viren ist ebenfalls nicht unproblematisch, da sie infektiöses Material darstellen und damit ein hoher Sicherheitsstandard eingehalten werden muss.

In der Veröffentlichung WO 94/19013 A wird eine Impfstoff-Zusammensetzung beschrieben, die ein hochgereinigtes antigenes Influenza-Polypeptid als Influenza-Antigen und 3D-MPL (3-o-Deacylated Monophosphoryl Lipid A) als Adjuvans enthält, und die eine effektivere Immunantwort in Mäusen induziert als ein derart hochgereinigtes Influenzavirus-Antigen allein. Gegenbenenfalls kann diese Zusammensetzung weitere Adjuvantien enthalten.

In der WO 96/40290 A werden immunologische Zusammensetzungen diverser bakterieller und viraler Antigene angeführt, die ein Lipoprotein enthalten und gegebenenfalls ein Adjuvans.

In der WO 97/49423 werden Influenza-Subunit-Vakzine enthaltend ein Influenza-Antigen und einen Cytokin-Immunpotentiator beschrieben, wobei beide vorzugsweise in ein Liposom eingebettet sind.

Aufgabe der vorliegenden Erfindung ist es daher, eine Influenzavirus-Vakzinzusammensetzung bereitzustellen, die die oben beschriebenen Nachteile nicht aufweist und die effektiv eine IgA- und IgG-Immunantwort bei Säugern induziert.

Die Aufgabe wird erfindungsgemäß gelöst durch die Verwendung einer Vakzin-Zusammensetzung, enthaltend mindestens ein inaktiviertes Influenzavirus oder Influenzavirus-Antigen und Aluminium als einziges Adjuvans zur nasalen oder oralen Verabreichung. Die beschriebene Zusammensetzung eignet sich insbesondere als Vakzine zur Prophylaxe gegen Influenzavirus-Infektionen.

Es hat sich im Rahmen der vorliegenden Erfindung gezeigt, dass eine inaktivierte Influenzavirus-Vakzine enthaltend Aluminium als Adjuvans, die nasal oder oral verabreicht wird, eine effektive IgG- sowie IgA-Immunantwort bei Säugern auslöst. Dies war insbesondere deshalb überraschend, da bei bisherigen Ansätzen zur Entwicklung von effektiven Influenzavirus-Vakzinen gefunden wurde, dass der adjuvantierende Effekt von Aluminium zur Erhöhung der Immunogenität des Antigens sogar bei einem parenteral verabreichtem Impfstoff sehr gering ist (Davenport et al., 1968, J. Immunol. 100:1139-1140).

Es wurde ferner gefunden, daß bei der erfindungsgemäßen nasalen oder oralen Verwendung der Vakzin-Zusammensetzung ein wesentlich höherer IgG- und IgA-Titer sowie ein höherer HAI-Titer in Säugern erreicht wird als bei bisher bekannten Impfstoff-Formulierungen, die entweder nur inaktivierte Influenzaviren, inaktivierte Viren mit Cholera-Toxin oder Lebendviren enthalten (Tabelle 1).

Die erfindungsgemäße Verwendung ist daher insbesondere zur Induktion einer protektiven mukosalen IgA- und einer systemischen IgG-Immunantwort geeignet.

Da Aluminium das einzige für die Anwendung am Menschen zugelassene Adjuvans ist, hat die erfindungsgemäße Verwendung der Impfstoff-Kombination von inaktiviertem Influenzavirus und Aluminium den großen Vorteil, daß sie unproblematisch für die direkte Verwendung beim Menschen ist. Der besondere Vorteil der erfindungsgemäßen Verwendung liegt daher neben der erhöhten Immunreaktivität der Vakzinzusammensetzung bei nasaler oder oraler Verabreichung auch darin, daß durch Verwendung eines Adjuvans, das über lange Jahre erprobt ist und seine Anwendung am Menschen zugelassen ist, die Vakzine vollständig nebenwirkungsfrei ist.

Für die erfindungsgemäße Verwendung kann die Zusammensetzung Aluminium vorzugsweise in Form von Aluminiumhydroxid (Al(OH)₃) oder Aluminiumphosphat (AlPO₄) enthalten. Die Konzentration des Aluminiums ist dabei in der Vakzine vorzugsweise in einer Endkonzentration von 0,05% bis 0,5%.

Die Influenzavirus-Antigenmenge in der Vakzine ist dabei die für eine Impfstoffdosis übliche Antigenmenge. Vorzugsweise liegt die Antigenmenge, die in einer Impfdosis enthalten ist, zwischen 1,5 µg Antigen/Dosis bis 50 µg Antigen/Dosis beim Menschen.

Das Influenzavirus-Antigen kann aus infizierten Eiern über konventionelle Methoden hergestellt und gereinigt werden.

Vorzugsweise wird das Virusantigen jedoch gewonnen aus einer infizierten Zellkultur, wie beispielsweise in WO 96/15231 beschrieben. Besonders bevorzugt wird für die erfindungsgemäße Verwendung zur Herstellung einer Influenzavirus-Vakzine ein Influenzavirus-Antigen, das aus einer in serum- und proteinfreiem Medium gezüchteten VERO-Zellkultur, infiziert mit Influenzavirus, gewonnen wird. Das aus der infizierten Zellkultur erhaltene Virusantigen wird zuerst mit Formalin inaktiviert und kann dann anschließend mittels kontinuierlicher Dichtezentrifugation im Gradienten, DNAse-Behandlung und Dia-und Sterilfiltration als gereinigte, konzentrierte Virusantigen-Präparation gewonnen werden. Diese konzentrierte Präparation kann dann zusammen mit Aluminium als Adjuvans für die erfindungsgemäße Verwendung zur Herstellung einer nasal und oral verabreichbaren Vakzine eingesetzt werden.

Ein besonderer Vorteil bei der Herstellung der Vakzine ist, daß das Virusmaterial vor der Reinigung inaktiviert wird, wodurch im Vergleich zur Reinigung von attenuierten Lebendviren eine wesentlich höhere Reinheit der Antigenpräpäration erreicht wird.

Ein besonderer Vorteil bei Verwendung von Influenzavirus-Antigen, gewonnen aus einer serum- und proteinfreien Zellkultur, infiziert mit Influenzavirus, ist die Abwesenheit von Ei-spezifischen Proteinen, die möglicherweise eine allergische Reaktion gegen diese Proteine auslösen können. Daher ist die erfindungsgemäße Verwendung für die Prophylaxe von Influenzavirus-Infektionen, insbesondere bei Personengruppen die zu einer Hochrisiko-Gruppe gehören, wie etwa Asthmatiker, Allergiker, aber auch Menschen mit Immunsuppression und bei älteren Menschen, geeignet.

Die Applikation der Vakzine kann dabei auf verschiedenen Wegen erfolgen.

Gemäß einer Ausführungsform der Erfindung erfolgt die Verabreichung über den mukosalen Weg intranasal. Die intranasale Verabreichung der Vakzinzusammensetzung kann dabei beispielsweise in flüssiger Form als Nasentropfen, Spray oder für die Inhalation geeignet, als Pulver, als Creme oder als Emulsionen formuliert sein.

Die Zusammensetzung kann verschiedene Zusätze wie beispielsweise Stabilisatoren, Puffer oder präservierende Mittel enthalten.

Vorzugsweise ist für die einfache Applikation die Vakzinzusammensetzung in einem entsprechenden Behältnis für die Verteilung des Antigens in Form von Nasentropfen oder eines Aerosols enthalten.

Gemäß einer weiteren Ausführungsform der Erfindung erfolgt die Verabreichung oral, wobei die Vakzine beispielsweise in Form einer Tablette, oder eingehüllt in eine Gelatinekapsel oder einer Mikrokapsel, vorliegen kann, wodurch die orale Applikation vereinfacht wird. Die Herstellung dieser Applikationsformen liegt im allgemeinen Wissen eines Fachmannes.

Die Erfindung wird anhand der nachfolgenden Beispiele näher erläutert, wobei sie nicht auf die Beispiele beschränkt ist.

### Beispiel 1:

### Herstellen einer Influenzavirus-Impfstoffpräparation

Influenzavirus wurde aus proteinfreier Vero-Zellkultur, infiziert mit Influenza A- oder B-Virusstamm, gemäß WO 96/15231 oder nach konventionellen Methoden aus Allantoisflüssigkeit von infizierten bebrüteten Hühnereiern gewonnen.

Zur Herstellung einer inaktivierten Influenzavirus-Präparation aus Zellkultur wurde der Überstand einer infizierten Vero-Zellkultur mit Formalin (Endkonzentration 0,025%) versetzt und die Viren bei 32°C für 24 Stunden inaktiviert. Dieses Material wurde durch zonale Zentrifugation in einem kontinuierlichen 0-50% Saccharose-Gradienten, DNAse-Behandlung, Diafiltration und Sterilfiltration gereinigt. Das gereinigte Material wurde bei -70°C gelagert. Das Endprodukt wurde auf Restkontaminationen getestet und pro Dosis wurden folgende Kriterien festgelegt:

| | |
|---|---|
| Hämagglutiningehalt: | ≥ 15 µg HA pro Stamm |
| Proteingehalt: | ≤ 250 µg |
| Saccharosegehalt: | ≤ 200 mg |
| Formalingehalt: | ≤ 5 µg |
| Benzonasegehalt: | ≤ 5 ng |
| Rest-DNA (VERO): | ≤ 100 pg |
| Endotoxingehalt: | ≤ 100 EU |
| Pyrogen: | frei |

### Beispiel 2:

### Intranasale Immunisierung von Mäusen mit verschiedenen Influenzavirus-Präparationen

Die Antigen-Präparationen aus Beispiel 1 wurden in PBS bis zu einem HA-Antigengehalt von 15 µg/ml verdünnt und gegebenenfalls mit Al(OH)₃ bis zu einer Endkonzentration von 0;2% bzw. mit Cholera-Toxin adjuvantiert. Zur Herstellung einer Präparation zur intranasalen Immunisierung von Mäusen wurde die Lösung auf die entsprechende Antigenmenge mit PBS, gegebenenfalls enthaltend Al(OH)₃ oder Choleratoxin, verdünnt.

Je 4 Balb/c-Mäuse wurden mit verschiedenen Influenzavirus-Präparationen intranasal immunisiert, wobei jeweils 50 µl einer Lösung, enthaltend Influenzavirus-Antigen und gegebenenfalls ein Adjuvans den Mäusen in die Nasenhöhlen getropft wurden. Die 1. Immunisierung erfolgte dabei am Tag 0, die 2. am Tag 7 und die 3. am Tag 14. Am Tag 28 wurden der IgG-, IgA- und HAI-Titer aus Serum, Speichel und Lungenspülung bestimmt.

Tabelle 1 zeigt dabei das Schema der intranasalen Immunisierung der einzelnen Mäusegruppen mit unterschiedlichen Influenzavirus-Präparationen.

**Tabelle 1**

| Impfschema der intranasalen Immunisierung von Mäusen | | | |
|---|---|---|---|
| **Gruppe Balb/c-Mäuse** | **Antigen** | **Dosis** | **Route** |
| | | | |
| 1. Gruppe | inaktivierte Ganzviren aus VERO-Zellen | 1 µg HA | 50 µl/i.n. |
| 2. Gruppe | inaktivierte Ganzviren aus infizierten Eiern | 1 µg HA | 50 µl/i.n. |
| | | | |
| 3. Gruppe | Lebendviren aus VERO-Zellen | 5 x 10⁶ EID₅₀ | 50 µl/i.n. |
| 4. Gruppe | Lebendviren aus infizierten Eiern | 5 x 10⁶ EID₅₀ | 50 µl/i.n. |
| | | | |
| 5. Gruppe | VERO-Mock-Präparation | 5% von 1 µg | 50 µl/i.n. |
| 6. Gruppe | Eier-Mock-Präparation | 5% von 1 µg | 50 µl/i.n. |

### Beispiel 3:

### Bestimmung des IgA-Titers im Speichel, Lungenlysat und Serum, des IgG-Titers und des HAI-Titers im Serum

Am Tag 28 nach Immunisierung wurden den Tieren aus Speichel, Lungen und Serum Proben entnommen und der Antikörper-Titer in den einzelnen Proben bestimmt.

Speichelproben wurde durch Injektion von 0,5 ml PBS in der Maus-Mundhöhle gesammelt und auf Anwesenheit von IgA-Antikörper getestet.

Zur Herstellung von Lungenlysatproben wurden die Mäuse getötet, die Brust geöffnet, die Lungen entnommen und mit PBS gespült. Anschließend wurden die Lungen zerkleinert und das Homogenat zentrifugiert, um Zellgewebe zu entfernen. Der Überstand wurde gesammelt und bis zur Austestung auf IgA-Antikörper bei -20°C aufbewahrt.

Der IgG- und IgA-Antikörpertiter wurde mittels eines kommerziellen Influenzavirus-Typ A und B ELISA-Test (Genzyme Virotech) bestimmt. Nach 2-stündiger Inkubation bei 37°C wurden die spezifischen IgG- und IgA-Antikörper mit konjugiertem Ziegen-anti-Maus-IgG oder -IgA (Pharmigen) und chromogenem Substrat, enthaltend H₂O₂ und O-Phenyldiamin, detektiert.

Zur Bestimmung des HAI-Titers wurde den Mäusen Blut entnommen und das erhaltene Serum im Influenza A- bzw. B- Hämagglutinationstest (HAI-Titer) nach der Methode von Palmer et al. (1975, Advanced laboratory technicals for immunological diagnostic, U.S. Dept. Health. Ed. Welfare. P.H.S. Atlanta, Immunology Ser. No. 6, Procedural guide, part 2, haemagglutination - inhibition test, S. 25-62) getestet.

Tabelle 2 zeigt eine Zusammenfassung der Bestimmung des IgA-Antikörpertiters im Speichel, Lungenlysat und Serum und des IgG-Antikörpertiters sowie HAI-Titers im Serum. Die Daten zeigen eindeutig, daß durch eine inaktivierte Ganzvirus-Vakzine ohne Adjuvans weder die IgA- noch IgG-Immunantwort stimuliert wird. Hingegen erfolgt bei der Immunisierung mit Lebendvirus oder inaktiviertem Ganzvirus adjuvantiert mit Cholera-Toxin sowohl ein Anstieg des IgA,- IgG- und HAI-Titers in den Mäusen, ebenso wie nach Immunisierung mit inaktiviertem Ganzvirus adjuvantiert mit Aluminium, wobei bei letzterem Impfstoff die IgG-Immunantwort im Serum sogar am höchsten von allen getesteten Präparationen war. Bei der inaktivierten Vakzine mit Aluminium wurde ebenfalls der höchste HAI-Titer gemessen.

Die Ergebnisse zeigen, daß die intranasale Immunisierung mit inaktivierter Influenzavirus-Vakzine adjuvantiert mit Aluminium im Vergleich zu bisher bekannten Influenzavirus-Impfstoffpräparationen eine leicht erhöhte IgA-Immunreaktion, und eine wesentlich höhere IgG-Immunantwort in Säugern induziert, ohne die Nachteile eines Lebendvirus-Impfstoffes bzw. eines Impfstoffes adjuvantiert mit einem nicht für die Anwendung beim Menschen zugelassenen Adjuvans, wie Cholera-Toxin, aufzuweisen.

### Beispiel 4:

### Lagerstabilität der Vakzinzusammensetzung bei verschiedenen Temperaturen

Für Stabilitätsuntersuchungen wurden die monovalenten Bulks (MB) der Influenzavirus Impfstämme Johannesburg 82, Nanchang und B/Harbin 12 Monate bei +4°C, -20°C und -80°C gelagert. Nach 6 und 12 Monaten wurde der spezifische Hämagglutinationstest (HA)-Gehalt der MB's Johannesburg 82 (MB/J/0197), Nanchang (MB/N/0197) und B/Harbin (MB/H/0397) ohne Pluronic sowie Johannesburg 82 (MB/J/0297/P), Nanchang (MB/N/0297P) und B/Harbin (MB/H/0497P) mit Pluronic mittels SRD (Single radial immunodiffusion)-Assay nach Wood et al., 1977, J. Biol. Standard 5: 237-247 bestimmt und die Abweichung vom Ausgangswert in Prozent berechnet.

Die trivalenten Bulks (TVB) 410197 (ohne Pluronic) und 4102997P (mit Pluronic) wurden ebenfalls 12 Monate bei +4°C gelagert und anschließend im SRD ausgetestet. Darüberhinaus wurden noch die Rückhalte-Muster dieser MB's und TVB's, die für Sterilitätsuntersuchungen bei Raumtemperatur gelagert wurden, nach 12 Monaten im SRD analysiert.

Die Ergebnisse der MB's sind in Tabelle 3 und die Ergebnisse der TVB's in Tabelle 4 dargestellt:

Die Lagerung der MB's bei +4°C und -80°C für 1 Jahr zeigt so gut wie keine Abnahme des spezifischen HA-Gehalts gegenüber dem Ausgangswert im Falle von Johannesburg 82 und Nanchang. Die B/Harbin-Präparationen scheinen weniger stabil zu sein, sie fallen um ca. 25% (ohne Pluronic) und ca. 40% (mit Pluronic) ab. Die Lagerung bei -20°C scheint einen signifikanten Einfluß auf die Stabilität zu haben, bei Johannesburg 82 fallen die Werte um 27% (ohne Pluronic) bzw. 11% (mit Pluronic), bei Nanchang um 9% (ohne Pluronic) bzw. 19% (mit Pluronic) und bei B/Harbin um 34% (ohne Pluronic) bzw. 47% (mit Pluronic). Die Ergebnisse der Lagerung bei Raumtemperatur zeigen eine erstaunliche Stabilität der Präparation. Bei Johannesburg 82 sind noch etwa 80% des ursprünglichen HA-Gehalts nachweisbar, bei Nanchang ca. 65% und bei B/Harbin ca. 45%. Die Lagerung der TVB's bei +4°C über 1 Jahr zeigt erneut keine signifikanten Unterschiede im HA-Gehalt. Die Stabilität der TVB's bei Raumtemperatur für 1 Jahr unterscheidet sich bei den 3 Stämmen: bei Johannesburg 82 gibt es keine signifikanten Unterschiede im HA-Gehalt, bei Nanchang nimmt er leicht ab (ca. 10%) und bei B/Harbin sinkt er um etwa ein Drittel.

Die Präparationen sind insgesamt sehr stabil, sogar bei einer Lagerung bei Raumtemperatur und es gibt keine signifikanten Unterschiede zwischen den Präparationen mit und ohne Pluronic.

**Tabelle 2**

| **intranasale Immunisierung von Mäusen mit verschiedenen Influenza-Präparationen** | | | | | | | | | | | | | |
|---|---|---|---|---|---|---|---|---|---|---|---|---|---|
| **und Bestimmung der IgA-Titers im Speichel, Lungenlysat und Serum, und des IgG-Titers sowie HAI-Titers im Serum** | | | | | | | | | | | | | |
| Immunogen | Stamm | Adjuvans | Titer | | | | | | | | | | |
| | | | IgA | | | | | | IgG | | HAI | | |
| | | | Speichel | | Lungenlysat | | Serum | | Serum | | Johann 82 A/H1N1 | Nanchang A/H3N2 | B/Harbin B |
| | | | Flu A | Flu B | Flu A | Flu B | Flu A | Flu B | Flu A | Flu B | | | |
| Vero-Vakzine (inaktiviert) | J, N, H | - | < 10 | < 10 | < 10 | < 10 | < 10 | < 10 | 800 | 100 | 80 | 80 | 20 |
| | | Al(OH)₃ | 40 | 10 | 320 | 40 | 160 | < 10 | 102.400 | 3.200 | 1.280 | 640 | 160 |
| | | CTB | 20 | 10 | n.b. | n.b. | 80 | < 10 | 51.200 | 12.800 | 640 | 640 | 160 |
| Egg-Vakzine (inaktiviert) | J, N, H | - | 10 | 10 | 10 | < 10 | < 10 | < 10 | 1.600 | 100 | 160 | 160 | 40 |
| | | Al(OH)₃ | 40 | 20 | 320 | 40 | 160 | 10 | 51.200 | 6.400 | 640 | 320 | 160 |
| Lebend-Virus Vero | N | - | 20 | < 10 | n.b. | n.b. | 160 | < 10 | 51.200 | < 10 | 80 | 640 | < 10 |
| Lebend-Virus Ei | N | - | 40 | < 10 | n.b. | n.b. | 160 | < 10 | 51.200 | < 10 | 80 | 320 | 20 |
| Vero Mock | - | - | <10 | <10 | <10 | <10 | <10 | <10 | <10 | <10 | 80 | 160 | 20 |
| | | Al(OH)₃ | < 10 | < 10 | n.b. | n.b. | < 10 | < 10 | < 10 | < 10 | 80 | 160 | < 10 |
| | | CTB | < 10 | < 10 | n.b. | n.b. | < 10 | < 10 | < 10 | < 10 | 80 | 160 | < 10 |
| Ei Mock | - | - | < 10 | < 10 | < 10 | < 10 | < 10 | < 10 | < 10 | < 10 | 80 | 160 | 20 |
| | - | Al(OH)₃ | < 10 | < 10 | < 10 | < 10 | < 10 | < 10 | < 10 | < 10 | 80 | 160 | 20 |

| | | | | | | | | | | | | | |
|---|---|---|---|---|---|---|---|---|---|---|---|---|---|
| J: Johannesburg 82 (A/H1N1), N: Nanchang (A/H3N2), H:B/Harbin, n.b.= nicht bestimmt | | | | | | | | | | | | | |

**Tabelle 3**

| **Lagerstabilität der MB's Influenza-Impfstoff der Saison 1997198 I** | | | | | |
|---|---|---|---|---|---|
| | **Lot** | **Lagerung** | **0 Monate** | **6 Monate** | **12 Monate** |
| Johannesburg 82 | | +4°C | | 204µg [111%] | 189µg [103%] |
| | MB/J/0197 | -20°C | 184µg | 182µg [99%] | 134µg [73%] |
| | | -80°C | | 210µg [114%] | 187µg [102%] |
| | | RT | | - | 152µg [83%] |
| | | +4°C | | 230µg [116%] | 207µg [105%] |
| | MB/J/0297P | -20°C | 198µg | 202µg [102%] | 177µg [89%] |
| | | -80°C | | 226µg [114%] | 212µg [107%] |
| | | RT | | - | 161µg [81%] |
| Nanchang | | +4°C | | 130µg [103%] | 131µg [104%] |
| | MB/N/0197 | -20°C | 126µg | 124µg [98%] | 115µg [91%] |
| | | -80°C | | 143µg [114%] | 132µg [105%] |
| | | RT | | - | 83µg [66%] |
| | | +4°C | | 128µg [91%] | 134µg [96%] |
| | MB/N/0297P | -20°C | 140µg | 139µg [99%] | 113µg [81%] |
| | | -80°C | | 143µg [102%] | 150µg [107%] |
| | | RT | | - | 90µg [64%] |
| B/Harbin | MB/H/0397 | +4°C | | 89µg [77%] | 83µg [72%] |
| | | -20°C | 116µg | 101µg [87%] | 76µg [66%] |
| | | -80°C | | 97µg [84%] | 88µg [76%] |
| | | RT | 324µg | - | 148µg [46%] |
| | MB/H/04/97P | +4°C | | 95µg [65%] | 87µg [60%] |
| | | -20°C | 146µg | 108µg [74%] | 77µg [53%] |
| | | -80°C | | 105µg [72%] | 89µg [61%] |
| | | RT | 374µg | - | 159µg [43%] |

| | | | | | |
|---|---|---|---|---|---|
| RT: Raumtemperatur P: mit Pluronic Angabe des spezifischen Hämagglutinations-(HA)Gehalt pro ml und in Klammem, Angabe ders HA-Gehalts zum Ausgangswert in Prozent | | | | | |

**Tabelle 4**

| **Lagerstabilität Influenza-Impfstoff der Saison 1997/98 II** | | | | | |
|---|---|---|---|---|---|
| **Lagerung der TVB's (Trivalenten Bulks) bei +4°C und bei Raumtemperatur** | | | | | |
| Stamm | Lot | Pluronic | Lagerung | | |
| | | | 0 Monate | 12 Monate | |
| | | | | +4°C | Raumtemperatur |
| Johannesburg 82 | 410198 | - | 16.8µg | 17,5µg [104%] | 15,8µg [94%] |
| Nanchang | 410198 | - | 15,9µg | 16,3µg [103%] | 14,1µg [89%] |
| B/Harbin | 410198 | - | 16,3µg | 14,1µg [87%] | 10,6µg [65%] |
| Johannesburg 82 | 410298P | + | 16,9µg | 17,4µg [103%] | 17,3µg [102%] |
| Nanchang | 410298P | + | 15,4µg | 13,9µg [90%] | 13,9µg [90%] |
| B/Harbin | 410298P | + | 14,5µg | 14,1µg [97%] | 9,7µg [67%] |

| | | | | | |
|---|---|---|---|---|---|
| Angabe des spezifischen Hämagglutinations-(HA) Gehalts pro Dosis (=0,5ml) und in Klammern Angabe der Änderung des HA-Gehalts zum Ausgangswert in Prozent | | | | | |

## Patentansprüche

1. Verwendung einer Zusammensetzung enthaltend inaktiviertes Influenzavirus und Aluminium als einziges Adjuvans zur Herstellung einer Influenzavirus-Vakzine zur nasalen oder oralen Verabreichung.

2. Verwendung nach Anspruch 1, **dadurch gekennzeichnet, dass** die Vakzine zur Prophylaxe von Influenzavirus-Infektionen geeignet ist.

3. Verwendung nach Anspruch 1 oder 2, **dadurch gekennzeichnet, dass** die Influenzavirus-Vakzine einen Virusantigengehalt zwischen 1,5 µg Antigen/Dosis und 50 µg Antigen/Dosis beim Menschen aufweist.

4. Verwendung nach einem der Ansprüche 1 bis 3, **dadurch gekennzeichnet, dass** die Vakzine für die Applikation in Form von Tropfen, eines Sprays oder für die Inhalation geeignet vorliegt.

5. Verwendung nach einem der Ansprüche 1 bis 3, **dadurch gekennzeichnet, dass** die Vakzine für die Applikation in Form von Tabletten, Gelatinekapsel oder Mikrokapsel vorliegt.

6. Verwendung nach Anspruch 1 oder 2, **dadurch gekennzeichnet, dass** die Vakzine für die Applikation in Form einer Creme oder Emulsion vorliegt.

7. Verfahren zur Herstellung einer Influenzavirus-Vakzine zur nasalen oder oralen Verabreichung, wie in einem der Ansprüche 1 bis 6 definiert, **dadurch gekennzeichnet, dass** ein inaktiviertes Influenzavirus mit Aluminium kombiniert wird und zu einem verabreichbaren Impfstoff formuliert wird.

8. Verfahren nach Anspruch 7, **dadurch gekennzeichnet, dass** das inaktivierte Influenzavirus aus einer infizierten Zellkultur gewonnen wird.

9. Verfahren nach Anspruch 7 oder 8, **dadurch gekennzeichnet, dass** das inaktivierte Influenzavirus aus einer serum- und proteinfreien Medium gezüchteten Zellkultur, infiziert mit Influenzavirus, gewonnen wird.

10. Verfahren nach einem der Ansprüche 7 bis 9, **dadurch gekennzeichnet, dass** das inaktivierte Influenzavirus nach dessen Inaktivierung einer Reinigung unterworfen wird.

## Claims

1. The use of a composition containing inactivated influenza virus and aluminium as the only adjuvant for preparing an influenza virus vaccine for nasal or oral administration.

2. The use according to claim 1, **characterised in that** the vaccine is suitable for the prophylaxis of influenza virus infections.

3. The use according to claim 1 or 2, **characterised in that** the influenza virus vaccine comprises a virus antigen content of between 1.5 µg of antigen/dose and 50 µg of antigen/dose in humans.

4. The use according to any one of claims 1 to 3, **characterised in that** the vaccine is provided suitable for the application in the form of drops, a spray, or for inhalation.

5. The use according to any one of claims 1 to 3, **characterised in that** the vaccine is provided for the application in the form of tablets, gelatine capsules or microcapsules.

6. The use according to claim 1 or 2, **characterised in that** the vaccine is provided for the application in the form of a cream or emulsion.

7. A method of producing an influenza vaccine for nasal or oral administration as defined in any one of claims 1 to 6, **characterised in that** an inactivated influenza virus is combined with aluminium and formulated to a vaccine to be administered.

8. The method according to claim 7, **characterised in that** the inactivated influenza virus is recovered from an infected cell culture.

9. The method according to claim 7 or 8, **characterised in that** the inactivated influenza virus is recovered from a cell culture grown in a medium that is free from serum and protease and that is infected with influenza virus.

10. The method according to any one of claims 7 to 9, **characterised in that** the inactivated influenza virus is subjected to a purification after having been inactivated.

## Revendications

1. Utilisation d'une composition comprenant un virus de la grippe inactivé et de l'aluminium comme adjuvant unique pour la production d'un vaccin contre le virus de la grippe, pour administration nasale ou orale.

2. Utilisation selon la revendication 1, **caractérisée en ce que** le vaccin est approprié pour la prophylaxie des infections au virus de la grippe.

3. Utilisation selon la revendication 1 ou 2, **caractérisée en ce que** le vaccin contre le virus de la grippe présente une teneur en antigène viral entre 1,5 µg d'antigène/dose et 50 µg d'antigène/dose chez l'être humain.

4. Utilisation selon l'une quelconque des revendications 1 à 3, **caractérisée en ce que** le vaccin se présente, pour l'application, sous forme de gouttes, d'un spray ou est approprié pour l'inhalation.

5. Utilisation selon l'une quelconque des revendications 1 à 3, **caractérisée en ce que** le vaccin se présente, pour l'application, sous la forme de comprimés, de capsules de gélatine et de microcapsules.

6. Utilisation selon la revendication 1 ou 2, **caractérisée en ce que** le vaccin se présente, pour l'application, sous la forme d'une crème ou d'une émulsion.

7. Procédé de production d'un vaccin contre le virus de la grippe pour administration nasale ou orale telle que définie dans l'une quelconque des revendications 1 à 6, **caractérisé en ce qu'**un virus de la grippe inactivé est combiné à l'aluminium et est formulé en vaccin pouvant être administré.

8. Procédé selon la revendication 7, **caractérisé en ce que** le virus de la grippe inactivé est obtenu à partir d'une culture cellulaire infectée.

9. Procédé selon la revendication 7 ou 8, **caractérisé en ce que** le virus de la grippe inactivé est obtenu à partir d'une culture cellulaire cultivée dans un milieu sans sérum et sans protéine, infecté avec le virus de la grippe.

10. Procédé selon l'une quelconque des revendications 7 à 9, **caractérisé en ce que** le virus de la grippe inactivé est soumis à une purification après son activation.
